# FASCICULE DE BREVET EUROPEEN

(11) **EP 0 678 276 B1**
(45) Date de publication et mention de la délivrance du brevet: **17.06.1998**
(21) Numéro de dépôt: 95400733.2
(22) Date de dépôt: 03.04.1995
(51) Int. Cl.: A61B 3/113, G02B 27/14

(54) **Dispositif de visée à suivi du regard**
Zielvorrichtung mit Blicknachfolge
Aiming device with view-following feature

(30) Priorité: 18.04.1994 FR 9404587
(43) Date de publication de la demande: 25.10.1995
(73) Titulaire: AEROSPATIALE Société Nationale Industrielle, 75781 Paris Cédex 16 (FR)
(72) Inventeur: Merle, Jean-Pierre, F-91400 Orsay (FR); Lassale, Martine, F-78960 Voisins le Bretonneux (FR); Bernoux, Frank, F-94240 L'Hay-les-Roses (FR); Adda, Maurice, F-92160 Antony (FR)
(74) Mandataire: Bonnetat, Christian

(56) Documents cités:
- EP-A- 0 042 812
- EP-A- 0 157 973
- DE-A- 2 322 017
- DE-B- 1 219 250
- DE-C- 900 021
- DE-C- 3 639 869
- FR-A- 2 685 099
- JOSA, vol. 48, no. 7, Juillet 1958 US, pages 439-445, J.F.MACKWORTH 'eye fixations recorded on changing visual scenes by the television eye marker'

## Description

La présente invention concerne un dispositif destiné à être associé, d'une part, avec une lunette de visée, du côté de l'oculaire de celle-ci, et, d'autre part, avec des moyens de calcul, pour former un système permettant de déterminer à chaque instant la portion d'un champ regardée par l'oeil d'un observateur à travers ladite lunette de visée.

On connaît déjà, par exemple par le brevet EP-A-0 157 973, un système destiné à déterminer la portion d'un champ regardée par l'oeil d'un observateur et comprenant :
- une source de lumière adressant un faisceau lumineux sur la cornée dudit oeil pour former un reflet cornéen ;
- des premiers moyens de prise de vue observant ledit reflet cornéen et engendrant des premiers signaux électriques représentatifs de l'orientation de l'axe optique dudit oeil ;
- des deuxièmes moyens de prise de vue observant ledit champ et engendrant des deuxièmes signaux électriques représentatifs dudit champ ; et
- des moyens de calcul recevant lesdits premiers et deuxièmes signaux électriques et déterminant, dans ledit champ, la portion de celui-ci regardée par ledit oeil.

Le fonctionnement d'un tel dispositif est basé notamment sur les enseignements contenus dans la publication de MACKWORTH et al., "Eye Fixations Recorded on Changing Visual Scenes by the Television Eye Marker" dans Journal of the Optical Society of America, juillet 1958, et montrant qu'il y a corrélation entre la variation en orientation de l'axe optique de l'oeil et le déplacement dudit reflet cornéen.

Par suite, en observant ledit reflet cornéen, lesdits premiers moyens de prise de vue connaissent, à chaque instant, la position du reflet cornéen et donc l'orientation de l'axe optique de l'oeil. Lesdits moyens de calcul peuvent donc déterminer la portion du champ regardée, à chaque instant, par ledit oeil, puisqu'ils reçoivent les informations de champ provenant des deuxièmes moyens de prise de vue.

Lesdits premiers et deuxièmes moyens de prise de vue peuvent être du type caméra, par exemple du type CCD et, de préférence, ladite source de lumière est invisible (infrarouge) pour ne pas gêner l'observateur. Elle est généralement du type diode LED.

Selon une variante de mise en oeuvre connue, on peut associer, audit reflet cornéen, le reflet rétinien engendré par ledit faisceau lumineux et diaphragmé par la pupille, afin de déterminer l'orientation de l'axe optique de l'oeil.

Dans la plupart des dispositifs connus, du type décrit ci-dessus, on prévoit un casque ou des lunettes, portés par ledit observateur et supportant au moins certains des éléments constitutifs (source de lumière, caméras, ...) du dispositif. Il en résulte une gêne et un manque de confort évidents pour l'observateur.

De plus, dans ces dispositifs connus à casque ou lunettes, l'oeil de l'observateur ne peut que regarder directement le champ, sans interposition d'un système optique, tel qu'une lunette de visée, limitant la vision périphérique. Il n'y a donc aucun obstacle à l'illumination de l'oeil par la source de lumière invisible.

En revanche, si l'on doit prévoir un système optique pour l'observation du champ par l'oeil de l'observateur, on se heurte à des difficultés.

En effet, l'oeil doit se trouver proche de l'oculaire du système optique, dans le plan de la pupille dudit système optique. Par suite, il n'est pas possible, faute de place, d'introduire le faisceau lumineux invisible entre l'oculaire du système optique et l'oeil. Par ailleurs, si l'on pensait illuminer l'oeil en lumière invisible à travers ledit système optique, le faisceau de lumière invisible serait limité en diamètre par la pupille dudit système optique. Etant donné que la surface de la pupille d'un système optique est petite par rapport à la surface de la cornée à illuminer, il en résulterait une impossibilité d'illuminer la totalité de la cornée et donc de mesurer toutes les orientations de l'axe optique de l'oeil.

Pour remédier à ces derniers inconvénients, le document FR-A-2 685 099 (91 15509) prévoit, en regard de sa figure 4, un dispositif placé à l'intérieur dudit système optique, au voisinage du foyer objet de son oculaire de sortie, ce dispositif comprenant :
- un élément optique séparateur disposé sur l'axe optique de ladite lunette de visée ;
- une source de lumière adressant sur ledit oeil, par l'intermédiaire dudit élément optique séparateur, un faisceau lumineux pour former un reflet ;
- des premiers moyens de prise de vue observant ledit reflet par l'intermédiaire dudit élément optique séparateur et engendrant des premiers signaux électriques représentatifs de l'orientation de l'axe optique dudit oeil, lesdits premiers signaux devant être adressés à des moyens de calcul ; et
- des deuxièmes moyens de prise de vue observant ledit champ par l'intermédiaire dudit élément optique séparateur et engendrant des deuxièmes signaux électriques représentatifs dudit champ, lesdits deuxièmes signaux devant également être adressés auxdits moyens de calcul.

Un tel dispositif, s'il résout les inconvénients précités, présente en revanche celui de nécessiter de le disposer à l'intérieur de la lunette de visée, ce qui soulève des difficultés de mise en place, d'ajustement et de réglage.

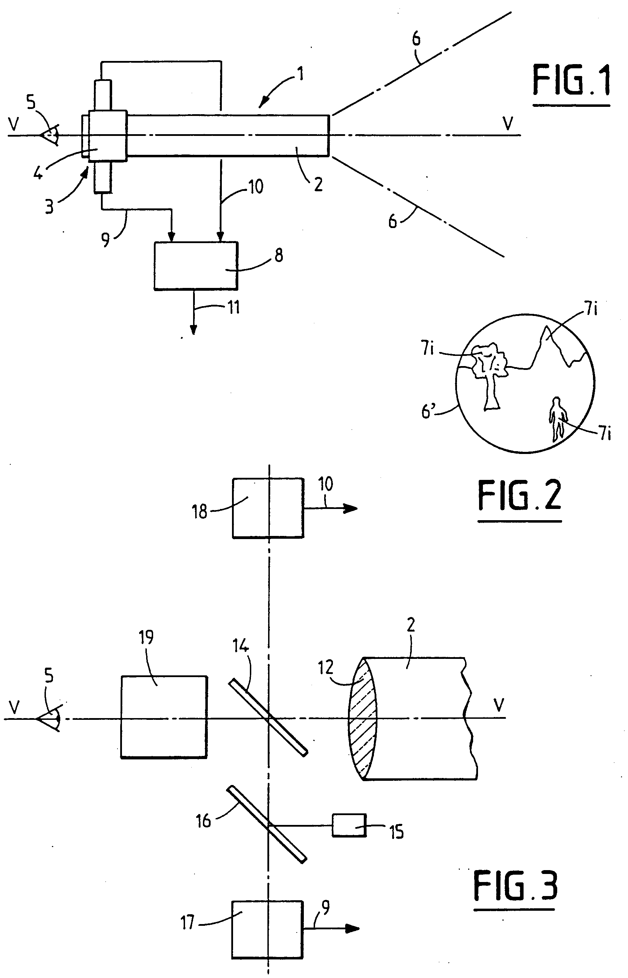

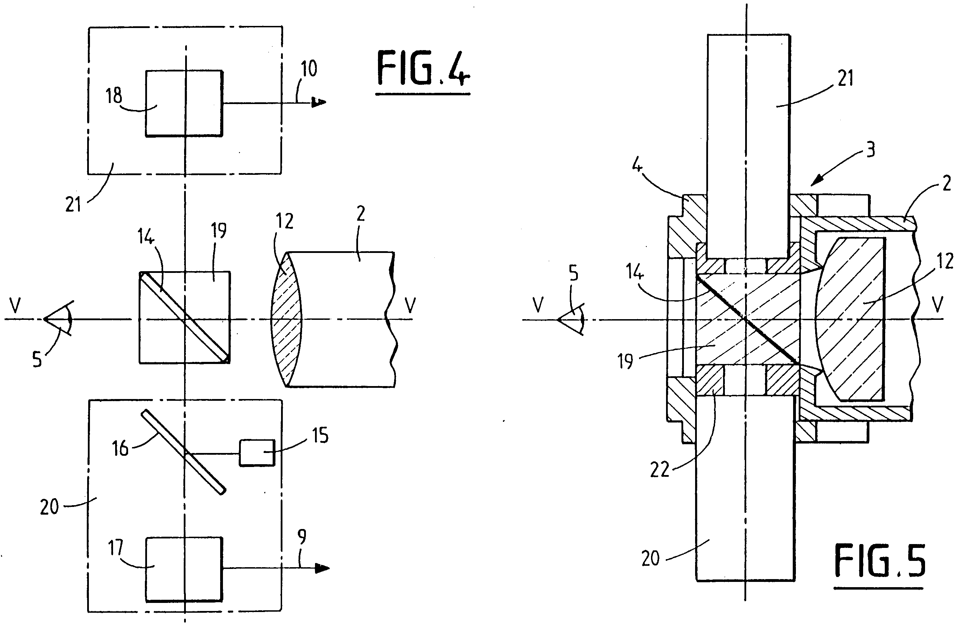

On remarquera que le document FR-A-2 685 099 mentionné ci-dessus prévoit en regard de sa figure 3 de placer, à l'extérieur de la lunette de visée, un dispositif différent de celui décrit en regard de la figure 4. Mais alors, il est prévu un véhicule optique afocal constitué d'une pluralité de lentilles. Un tel véhicule optique est complexe et source de reflets parasites, renvoyés par réflexion de lentilles en lentilles, susceptibles de perturber le bon fonctionnement du dispositif.

La présente invention a pour objet de remédier à ces inconvénients.

A cette fin, selon l'invention, le dispositif du type décrit ci-dessus, disposé du côté image de l'oculaire de ladite lunette de visée (c'est-à-dire à l'extérieur de celle-ci), est caractérisé en ce qu'il comporte une lame optique épaisse, dont les faces parallèles sont orthogonales audit axe optique de ladite lunette.

Ainsi, grâce à ladite lame optique épaisse, on obtient un dispositif simple, sans réglage et exempt de reflets parasistes et on éloigne la pupille de sortie de la lunette de visée de l'oculaire de celle-ci. On ménage donc suffisamment de place en aval de l'oculaire de la lunette pour loger ledit dispositif entre l'oeil et ledit oculaire. Ladite lame optique épaisse peut être disposée entre ledit élément optique séparateur et l'oeil, ou bien entre l'oculaire de la lunette de visée et ledit élément optique séparateur.

On notera que le fait de placer une lame optique épaisse du côté de image de l'oculaire d'un système optique est connu du document DE-C-900021 ; selon ce document, la lame ne reste cependant pas en permanence dans l'axe optique du système, mais est interposée pendant une fraction du temps d'exposition dans le cas d'un système photographique. Ce document ne suggère donc pas de modifier pour le simplifier le mode de réalisation de la figure 3 selon le document FR-A-2 685 099, en remplaçant le véhicule optique afocal constitué de quatre lentilles convergentes par une lame épaisse.

De préférence, dans le dispositif conforme à la présente invention, afin que l'éloignement de la pupille de sortie soit suffisant sans nécessiter une épaisseur trop importante pour la lame à face parallèle, il est avantageux que l'indice de réfraction de celle-ci soit grand, par exemple voisin de 2.

Dans un mode avantageux de réalisation, ladite lame optique épaisse présente la forme au moins approximative d'un cube.

Ledit élément optique séparateur peut être du type lame parallèle mince à surfaces traitées de façon à être, d'un côté, partiellement transparent et partiellement réfléchissant pour les rayons provenant dudit champ et, de l'autre côté, au moins partiellement réfléchissant pour ledit faisceau lumineux émis par ladite source de lumière.

Pour gagner encore de la place en aval de l'oculaire de la lunette de visée, il est alors avantageux que ledit élément optique séparateur soit incorporé dans ledit cube, selon un plan diagonal de celui-ci.

De préférence, ledit dispositif forme une unité de construction rapportée de façon amovible sur ladite lunette de visée.

On remarquera qu'un tel mode de réalisation est particulièrement avantageux, car il permet d'adapter ladite lunette de visée, sans modification de celle-ci, pour l'utiliser avec le dispositif conforme à la présente invention. On obtient ainsi un dispositif destiné à être couplé à une lunette de visée usuelle pour la rendre apte à la visée avec suivi du regard.

Cependant, il va de soi que le dispositif selon l'invention pourrait être incorporé à la lunette de visée, en aval de l'oculaire. Même dans ce cas, il n'y aurait aucune modification interne de la lunette de visée.

Dans un mode avantageux de réalisation, le dispositif de l'invention comporte une monture rendant solidaires le cube pourvu de l'élément séparateur et les premiers et deuxièmes moyens de prise de vue, ainsi que des moyens pour fixer ladite monture sur ladite lunette de visée.

Les figures du dessin annexé feront bien comprendre comment l'invention peut être réalisée. Sur ces figures, des références identiques désignent des éléments semblables.

La figure 1 montre schématiquement un mode de réalisation du dispositif conforme à la présente invention.

La figure 2 montre schématiquement l'image d'un champ vu à travers le dispositif de l'invention.

La figure 3 est une vue illustrant la constitution d'un premier exemple de réalisation du dispositif de la figure 1.

La figure 4 est une vue illustrant la constitution d'un second exemple de réalisation du dispositif de la figure 1.

La figure 5 illustre en coupe partielle un mode de réalisation pratique du second exemple du dispositif conforme à la présente invention.

Le système 1, conforme à la présente invention et représenté schématiquement sur la figure 1, comporte une lunette de visée 2, par exemple du type lunette astronomique, et un dispositif optique-électrique 3, solidarisés l'un de l'autre du côté de l'oculaire de ladite lunette par un élément de fixation 4, de façon que leurs axes optiques soient rigoureusement alignés sur un axe optique commun définissant une ligne de visée V-V.

Un oeil 5 d'un observateur est disposé sur la ligne de visée V-V, derrière le dispositif 3, de sorte qu'il peut regarder le champ 6 défini par la lunette de visée 2. Comme l'illustre la figure 2, l'oeil 5 voit donc l'image 6' du champ 6 à travers la lunette 2 et le dispositif 3, ainsi que les images des différents éléments ou portions 7i composant ce champ.

Par ailleurs, le système 1 de la figure 1 comporte un calculateur 8 recevant des signaux électriques de sorties 9 et 10 du dispositif 3 et les traitant pour délivrer des signaux à sa sortie 11.

Sur la figure schématique 3, la lunette 2 n'est représentée que partiellement.

La lunette 2 comporte un oculaire 12 et l'oeil 5 est disposé derrière cet oculaire pour recevoir le faisceau optique traversant ladite lunette (en provenance de l'objectif non représenté de ladite lunette 2) et pour voir l'image 6' (figure 2) du champ 6.

Entre l'oeil 5 et l'oculaire 12, est disposée une lame inclinée 14, qui, d'un côté, est partiellement transparente et partiellement réfléchissante pour le faisceau optique traversant la lunette, et, de l'autre côté, au moins partiellement réfléchissante pour la lumière infrarouge.

Par ailleurs, le dispositif de la figure 3 comporte :
- une source de lumière infrarouge 15 (diode LED), émettant un faisceau infrarouge, qui, par l'action d'un miroir incliné semi-transparent 16, est dirigé sur la lame inclinée 14 ;
- une caméra CCD infrarouge 17, disposée en regard de la lame inclinée 14, la sortie de la caméra 17 formant la sortie 9 de la lunette 2 ;
- une caméra CCD, disposée en regard de la lame inclinée 14, la sortie de la caméra 18 formant la sortie 10 de la lunette 2 ; et
- un cube optique 19, d'indice de réfraction élevé (voisin de 2), disposé sur la ligne de visée V-V avec deux de ses faces opposées orthogonales à ladite ligne de visée. Ledit cube optique 19 peut être placé entre l'oeil 5 et la lame inclinée 14 (comme représenté sur la figure 3), mais il peut également être disposé entre ladite lame inclinée 14 et l'oculaire 12.

Ainsi, le faisceau optique traversant la lunette de visée 2 est adressé à l'oeil 5 à travers l'oculaire 12, la lame inclinée 14 et le cube 19. De plus, une partie dudit faisceau optique est adressée à la caméra 18 par réflexion sur la lame inclinée 14. Les signaux électriques apparaissant à la sortie 10 représentent donc l'image 6' du champ 6.

Le faisceau infrarouge émis par la source 15 est adressé à l'oeil 5 après réflexion sur le miroir semi-transparent 16 et sur la lame inclinée 14, puis traversée du cube 19. Ce faisceau infrarouge engendre donc, par illumination de la cornée, de la rétine et/ou de la pupille de l'oeil 5 et par réflexion sur celles-ci, un reflet cornéen, rétinien et/ou pupillaire véhiculés par un faisceau infrarouge réfléchi, qui traverse, en sens inverse du faisceau infrarouge d'illumination, le cube 19, se réfléchit sur la lame inclinée 14 et traverse le miroir semi-transparent 16, avant d'être adressé à la caméra 17. Les signaux électriques apparaissant à la sortie 9 représentent donc le reflet cornéen et/ou rétinien.

Par suite, le calculateur 8 recevant à ses entrées les signaux électriques provenant des sorties 9 et 10 des caméras 17 et 18 est capable de délivrer à sa sortie 11, par un traitement connu desdits signaux électriques d'entrée, des signaux électriques de sortie représentatifs dudit élément 7i regardé par l'oeil 5.

De la description ci-dessus, on remarquera qu'il est aisé de réaliser, grâce à l'invention, un dispositif binoculaire en associant un système optique 2,3 à chacun des deux yeux 5 d'un observateur. Dans ce cas, il est avantageux que le calculateur 8 traite les signaux provenant des deux systèmes optiques 2,3.

Dans l'exemple de réalisation de la figure 4, on retrouve les différents éléments 14 à 19 de la figure 3. Cependant, dans ce cas, la lame inclinée 14 est incorporée dans le cube optique 19, selon un plan diagonal de celui-ci.

Comme le montre la figure 5, dans un mode de réalisation pratique de l'exemple de la figure 4, on prévoit :
- un boîtier 20 enfermant la caméra 17, la source infrarouge 15 et le miroir semi-transparent 16 ;
- un boîtier 21 enfermant la caméra 18 ;
- une monture 22 solidarisant le cube 19 (avec la lame 14) et les boîtiers 20 et 21 pour former le dispositif 3 ; et
- un dispositif de fixation 4 permettant de solidariser le dispositif 3 sur la lunette de visée 2, du côté image (ou extérieur) de l'oculaire 12.

Bien entendu, le dispositif de fixation 4 peut être amovible ou définitif.

## Revendications

1. Dispositif (3) destiné à être associé, d'une part, avec une lunette de visée (2), du côté image de l'oculaire (12) de celle-ci, et, d'autre part, avec des moyens de calcul (8), pour former un système permettant de déterminer à chaque instant la portion (7i) d'un champ (6) regardée par l'oeil (5) d'un observateur à travers ladite lunette de visée, ledit dispositif comprenant :
- un élément optique séparateur (14) disposé sur l'axe optique (V-V) de ladite lunette de visée (2) ;
- une source de lumière (15) adressant sur ledit oeil (5), par l'intermédiaire dudit élément optique séparateur (14) un faisceau lumineux pour former un reflet ;
- des premiers moyens de prise de vue (17) observant ledit reflet par l'intermédiaire dudit élément optique séparateur (14) et engendrant des premiers signaux électriques représentatifs de l'orientation de l'axe optique dudit oeil (5), lesdits premiers signaux devant être adressés auxdits moyens de calcul (8) ; et
- des deuxièmes moyens de prise de vue (18) observant ledit champ (6) par l'intermédiaire dudit élément optique séparateur (14) et engendrant des deuxièmes signaux électriques représentatifs dudit champ, lesdits deuxièmes signaux devant également être adressés auxdits moyens de calcul (8),
caractérisé en ce que ledit dispositif (3) comporte une lame optique épaisse (19), dont les faces parallèles sont orthogonales audit axe optique (V-V) de ladite lunette (2).

2. Dispositif selon la revendication 1,
caractérisé en ce que l'indice de réfraction de ladite lame optique épaisse (19) est voisin de 2.

3. Dispositif selon l'une des revendications 1 ou 2,
caractérisé en ce que ladite lame optique épaisse (19) présente la forme au moins approximative d'un cube.

4. Dispositif selon l'une quelconque des revendications 1 à 3,
caractérisé en ce que ledit élément optique séparateur (14) est une lame mince à surfaces traitées.

5. Dispositif selon la revendication 4,
caractérisé en ce que ledit élément optique séparateur (14) est, d'un côté, partiellement transparent et partiellement réfléchissant pour les rayons lumineux provenant dudit champ et, de l'autre côté, au moins partiellement réfléchissant pour ledit faisceau lumineux émis par ladite source de lumière.

6. Dispositif selon l'une des revendications 3 à 5,
caractérisé en ce que ledit élément optique séparateur (14) est incorporé dans ledit cube (19), selon un plan diagonal de celui-ci.

7. Dispositif selon l'une quelconque des revendications 1 à 6,
caractérisé en ce qu'il forme une unité de construction rapportable de façon amovible sur ladite lunette de visée (2).

8. Dispositif selon l'une quelconque des revendications 1 à 6,
caractérisé en ce qu'il est solidarisé à demeure de ladite lunette de visée (2).

9. Dispositif selon l'une quelconque des revendications 6 à 8,
caractérisé en ce qu'il comporte une monture (22) rendant solidaires le cube (19) pourvu de l'élément séparateur (14) et les premiers et deuxièmes moyens de prise de vue, ainsi que des moyens (4) pour fixer ladite monture sur ladite lunette de visée (2).

## Claims

1. Device (3) intended to be associated, on the one hand, with a viewfinder (2), on the image side of the eyepiece (12) thereof, and, on the other hand, with calculating means (8), to form a system making it possible to determine at every instant that portion (7i) of a field (6) sighted by the eye (5) of an observer through said viewfinder, said device comprising:
- an optical splitter element (14) arranged on the optical axis (V-V) of said viewfinder (2);
- a light source (15) sending a light beam to said eye (5), by way of said optical splitter element (14), in order to form a reflection;
- first picture-taking means (17) observing said reflection by way of said optical splitter element (14) and generating first electrical signals representative of the orientation of the optical axis of said eye (5), said first signals being required to be sent to said calculating means (8); and
- second picture-taking means (18) observing said field (6) by way of said optical splitter element (14) and generating second electrical signals representative of said field, said second signals likewise being required to be sent to said calculating means (8),
characterized in that said device (3) includes a thick optical plate (19) whose parallel faces are orthogonal to said optical axis (V-V) of said finder (2).

2. Device according to Claim 1, characterized in that the refractive index of said thick optical plate (19) is around 2.

3. Device according to either of Claims 1 and 2, characterized in that said thick optical plate (19) has the shape at least approximately of a cube.

4. Device according to any one of Claims 1 to 3, characterized in that said optical splitter element (14) is a thin plate with treated surfaces.

5. Device according to Claim 4, characterized in that said optical splitter element (14) is, on one side, partially transparent and partially reflecting in respect of the light rays originating from said field and, on the other side, at least partially reflecting in respect of said light beam emitted by said light source.

6. Device according to one of Claims 3 to 5, characterized in that said optical splitter element (14) is incorporated into said cube (19), along a diagonal plane thereof.

7. Device according to any of Claims 1 to 6, characterized in that it forms a construction unit which can be removably attached to said viewfinder (2).

8. Device according to any one of Claims 1 to 6, characterized in that it is solidly secured to said viewfinder (2).

9. Device according to any one of Claims 6 to 8, characterized in that it includes a mount (22) securing together the cube (19) provided with the splitter element (14) and the first and second picture-taking means, and also means (4) for fixing said mount onto said viewfinder (2).

## Patentansprüche

1. Vorrichtung (3), die einerseits mit einem Visierfernrohr (2) auf der Bildseite des Okulars (12) desselben und andererseits mit Rechenmitteln (8) verbunden wird und ein System bildet, durch das zu jedem Zeitpunkt der Abschnitt (7i) eines vom Auge (5) eines Beobachters durch das Visierfernrohr gesehenen Gesichtsfeldes (6) bestimmt werden kann, wobei die Vorrichtung umfaßt:
- ein Strahlenteilungselement (14) in der optischen Achse (V-V) des Visierfernrohrs (2);
- eine Lichtquelle (15), die an das Auge (5) über das Strahlenteilungselement (14) ein Lichtbündel zur Reflexbildung gibt;
- erste Aufnahmemittel (17), die den Reflex über das Strahlenteilungselement (14) beobachten und erste elektrische Signale erzeugen, die für die Richtung der optischen Achse des Auges (5) repräsentativ sind, wobei die ersten Signale an die Rechenmittel (8) gegeben werden müssen; und
- zweite Aufnahmemittel (18), die das Gesichtsfeld (6) über das Strahlenteilungselement (14) beobachten und zweite elektrische Signale erzeugen, die für das Gesichtsfeld repräsentativ sind, wobei die zweiten Signale ebenfalls an die Rechenmittel (8) zu geben sind,
dadurch gekennzeichnet, daß die Vorrichtung (3) eine dicke optische Platte (19) hat, deren planparallele Flächen senkrecht zur optischen Achse (V-V) des Fernrohrs (2) verlaufen.

2. Vorrichtung nach Anspruch 1,
dadurch gekennzeichnet, daß der Brechungsindex der dicken optischen Platte (19) in der Nähe von 2 liegt.

3. Vorrichtung nach einem der Ansprüche 1 oder 2,
dadurch gekennzeichnet, daß die dicke optische Platte (19) zumindest annähernd die Form eines Würfels hat.

4. Vorrichtung nach einem der Ansprüche 1 bis 3,
dadurch gekennzeichnet, daß das Strahlungsteilungselement (14) eine dünne Platte mit behandelten Oberflächen ist.

5. Vorrichtung nach Anspruch 4,
dadurch gekennzeichnet, daß das Strahlenteilungselement (14) auf der einen Seite für die Lichtstrahlen des Gesichtsfeldes teildurchlässig und teilreflektierend und auf der anderen Seite für das Lichtbündel der Lichtquelle zumindest teilreflektierend ist.

6. Vorrichtung nach einem der Ansprüche 3 bis 5,
dadurch gekennzeichnet, daß das Strahlenteilungselement (14) in den Würfel (19) in einer diagonalen Ebene desselben eingebaut ist.

7. Vorrichtung nach einem der Ansprüche 1 bis 6,
dadurch gekennzeichnet, daß sie eine Baueinheit bildet, die abnehmbar auf das Visierfernrohr (2) aufgesetzt werden kann.

8. Vorrichtung nach einem der Ansprüche 1 bis 6,
dadurch gekennzeichnet, daß sie dauerhaft mit dem Visierfernrohr (2) verbunden ist.

9. Vorrichtung nach einem der Ansprüche 6 bis 8,
dadurch gekennzeichnet, daß sie eine Fassung (22), durch die der Würfel (19) mit dem Strahlenteilungselement (14) und die ersten und zweiten Aufnahmemittel miteinander verbunden werden, sowie Mittel (4) zur Befestigung der Fassung am Visierfernrohr (2) hat.
